# EUROPEAN PATENT APPLICATION

(11) **EP 4 342 472 A1**
(43) Date of publication of application: **27.03.2024**
(21) Application number: 22804676.9
(22) Date of filing: 17.05.2022
(51) Int. Cl.: A61K 31/551, A61K 9/08, A61P 27/10, C07D 239/95

(54) **EYE DROP FOR SUPPRESSING MYOPIA**

(30) Priority: 17.05.2021 JP 2021082858
(71) Applicant: Tsubota Laboratory, Inc., Tokyo 160-0016 (JP)
(72) Inventor: JEONG, Heonuk, Tokyo 160-0016 (JP); KURIHARA, Toshihide, Tokyo 160-0016 (JP); TSUBOTA, Kazuo, Tokyo 160-0016 (JP)
(74) Representative: Mewburn Ellis LLP
(86) International application number: PCT/JP2022/020514
(87) International publication number: WO 2022/244765

(57) **Abstract**

To provide a new ophthalmic preparation for myopia suppression.

The above-described problem is solved by using an ophthalmic preparation containing bunazosin as an ophthalmic preparation for myopia suppression. This ophthalmic preparation for myopia suppression is effective for myopia suppression.

## Description

### Field of the Invention

The present invention relates to an ophthalmic preparation for myopia suppression.

### BACKGROUND ART

Myopia is particularly common among Asians, especially Japanese, and the percentage of those with severe myopia of -5D or greater is high as well. Further, in recent years, myopia has been on the rise worldwide and, in Japan as well, the percentage of children with naked eyesight of less than 1.0 has been increasing year by year. Furthermore, it is also known that myopia progresses rapidly during the school-age period from seven years old to twelve years old (refer to Non-Patent Document 1).

Myopia is classified into refractive myopia, accommodative myopia (pseudomyopia), and axial myopia in accordance with a mechanism of onset, but axial myopia is the main cause of myopia progression in the school-age period. The human eye is farsighted immediately after birth, and a degree of hyperopia decreases with axial elongation during a growth period, emmetropizing upon entry into the school-age period. Axial elongation after this emmetropization leads directly to myopia, and once elongated, the axial length cannot be returned to its original length. Accordingly, suppression of axial elongation during the growth period to the school-age period is considered effective for the prevention or treatment of myopia (refer to Non-Patent Document 2). Further, the eye axis is elongated not only during the growth period but also in an adult period, and axial elongation in the adult period is said to be a risk factor for various eye diseases.

Therapeutic agents have been proposed to prevent myopia, such as Rho kinase inhibitors (refer to Patent Document 1), bradykinin (refer to Patent Document 2), and crocetin (refer to Patent Document 3), for example.

### PRIOR ART DOCUMENTS

### Non-Patent Documents

Non-Patent Document 1: Igaku no ayumi (Journal of clinical and experimental medicine), Vol. 253, No. 2 (2015)
Non-Patent Document 2: Ophthalmology, Vol. 58, No. 6, 635-641 (2016)

### Patent Documents

Patent Document 1: International Publication WO2010/010702
Patent Document 2: Japanese Laid-Open Patent Application Publication No. 2011-144111
Patent Document 3: International Publication WO2018/212152

### SUMMARY OF THE INVENTION

### Problems to be Solved by the Invention

An object of the present invention is to provide a new ophthalmic preparation for myopia suppression.

### Means for Solving the Problems

An ophthalmic preparation for myopia suppression according to the present invention is an ophthalmic preparation containing bunazosin.

### Effect of the Invention

According to the present invention, it is possible to provide new ophthalmic preparations for myopia suppression that have demonstrated myopia suppression.

### BRIEF DESCRIPTION OF THE DRAWINGS

Fig. 1 is a photograph of a mouse used in a myopia induction experiment (-30D lens mounted on right eye and 0D on left eye).
Fig. 2 shows results showing a change in refractive index due to bunazosin.
Fig. 3 shows results showing a change in axial length due to bunazosin.
Fig. 4 shows results showing a change in refractive index due to unoprostone.
Fig. 5 shows results showing a change in axial length due to unoprostone.

### Embodiments of the Invention

An ophthalmic preparation for myopia suppression according to the present invention will now be described. The present invention is not limited to the contents of the following embodiments and examples, and includes various modifications and applications within the scope of the gist of the present invention.

### [Ophthalmic Preparation for Myopia Suppression]

The present inventors, with a focus on the fact that certain glaucoma ophthalmic preparations are effective in suppressing myopia progression, attempted to find myopia progression suppressing effects from existing glaucoma ophthalmic preparations. In the present application, glaucoma ophthalmic preparations were administered to a mouse myopia model, and an axial length and a refractive index were measured to evaluate whether the eye drops were effective in suppressing the progression of axial myopia.

As shown in experiment results described below, when ophthalmic solutions including the main ingredients of glaucoma ophthalmic preparations were evaluated, an ophthalmic solution containing bunazosin (refer to Experiment 1) exhibited a myopia-suppressing effect, but an ophthalmic solution containing unoprostone (refer to Experiment 2) did not exhibit a myopia suppressing effect. Glaucoma ophthalmic preparations are known to have the medicinal effect of lowering intraocular pressure and improving blood flow by promoting the excretion of aqueous humor. Although both bunazosin and unoprostone are ophthalmic preparations for glaucoma, it was found that, just because the preparations are ophthalmic preparations for glaucoma, a myopia suppressing effect cannot be expected.

In the present invention, it was discovered that an ophthalmic preparation containing bunazosin is effective as an ophthalmic preparation for myopia suppression.

The type of ophthalmic preparation containing bunazosin is not particular limited, but preferably is an aqueous ophthalmic preparation. In addition to bunazosin, other active ingredients (pharmacologically active ingredients, physiologically active ingredients, other glaucoma ophthalmic preparation ingredients, and the like) may be blended into the ophthalmic preparation, within a range that does not impair the effects of the present invention. The ophthalmic preparation can further contain one or more ingredients in combination, selected as appropriate from various ingredients and additives in accordance with usual methods, corresponding to application and form, within a range that does not impair the effects of the present invention. Examples of these ingredients or additives include various additives such as flavoring or refreshing agents, preservatives, fungicides or antibacterial agents, pH regulators, chelating agents, stabilizers, isotonizing agents, and buffering agents.

A content of bunazosin is set to a realistic blending quantity and, although 1 mL of Detantol 0.01% ophthalmic solution contains 0.1 mg of bunazosin hydrochloride in the experiment examples described below, preferably the content is about the same, and bunazosin can be blended within a range that does not impair the effects of the present invention. An application and a dosage of the ophthalmic preparation vary depending on the patient's symptoms, age, and other factors, but usually administration of about one to two drops each, about one to six times per day, is sufficient.

As described above, the present invention applies an ophthalmic preparation containing bunazosin as an ophthalmic preparation for myopia suppression, but can include the followings: (i) an ophthalmic preparation for myopia suppression, the ophthalmic preparation comprising a composition containing bunazosin; (ii) a method of manufacturing an ophthalmic preparation for myopia suppression, the method comprising using a composition containing bunazosin; and (iii) a method of suppressing myopia, the method comprising administering a composition containing bunazosin as an ophthalmic preparation. It should be noted that a "myopia suppressing effect" in the present application refers to an effect of suppressing the progression of axial myopia.

### Examples

The present invention will be described in further detail below using experimental examples.

### [Experiment 1]

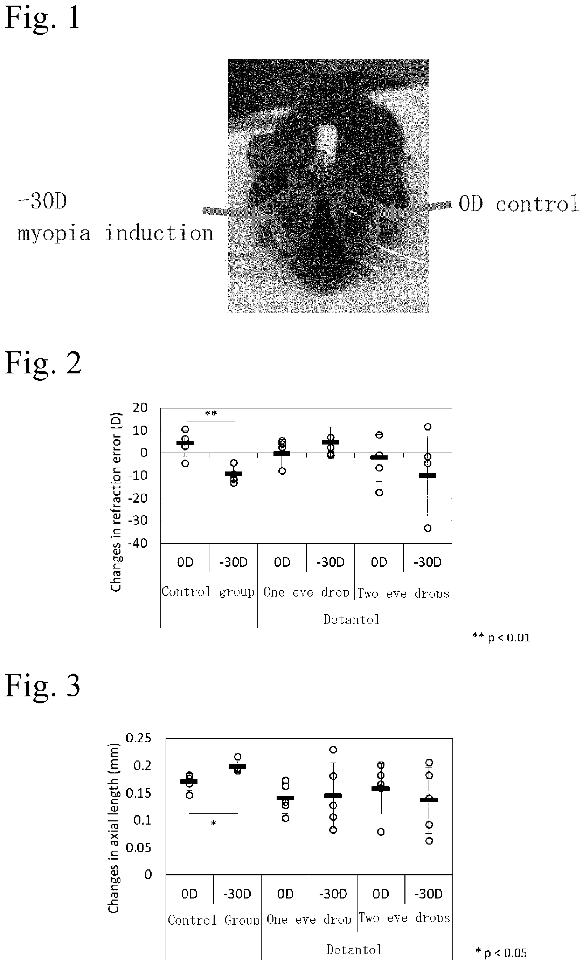

As shown in Fig. 1, a 0D (D is an abbreviation for diopter and is a unit of refractive power of a lens) lens and a -30D lens were mounted onto left eyes and right eyes, respectively, of three-week-old mice (C57BL6/J, Clea Japan, Inc.) to induce myopia. Eye drops were continuously administered for three weeks, with phosphate buffered saline (PBS) eye drops as the control group and bunazosin eye drops as the test group. Bunazosin is the main ingredient of a Detantol ophthalmic solution, and bunazosin eye drops were administered using Detantol 0.01% ophthalmic solution (manufactured by Santen Pharmaceutical Co. Ltd., 1 mL contains 0.1 mg of bunazosin hydrochloride). The number of eye drops was two drops once per day and twice a day (two drops each in the morning and evening). The refractive index and the axial length of the mouse eyeball after three weeks were measured and the amounts of change were calculated. It should be noted that, in the graph, one asterisk (*) indicates that p < 0.05 and two asterisks (**) indicate that p < 0.01 (the same in the graphs of the present application).

### (Measurement of refractive index and axial length)

The refractive values were measured by using an infrared photorefractor for mice (manufactured by Prof. Schaeffel, University of Tubingen). The axial lengths were measured by using spectral domain optical coherence tomography (Envisu R4310, manufactured by Leica).

### (Results)

Fig. 2 shows the refractive index results. The more negative the refractive index, the more severe the myopia. As the changes in refractive index in the control group, it was confirmed that the eyes with the -30D lenses became myopic and were significantly different from the eyes with 0D lenses. On the other hand, in the eye drop group, it was found that there was no significant difference between the eyes with the -30D lenses and the eyes with 0D lenses, and that the eye drops were effective in suppressing myopia. It should be noted that it was confirmed that one eye drop has a greater myopia suppressing effect than two eye drops.

Fig. 3 shows the axial length results. The greater the value of the axial length, the greater the severity of myopia. As the changes in axial length in the control group, it was confirmed that the axial lengths of the eyes with the -30D lenses elongated and were significantly different from the axial lengths of the eyes with 0D lenses. On the other hand, in the eye drop group, there was substantially no difference in the axial lengths between the two eyes, and a myopia suppressing effect was confirmed. It should be noted that there was no difference between one eye drop and two eye drops.

From the above results, it was confirmed that an ophthalmic solution containing bunazosin exhibits a myopia suppressing effect.

### [Experiment 2]

Mice similar to those in Experiment 1 were used to induce myopia. Eye drops were continually administered for three weeks, with PBS eye drops as the control group and unoprostone eye drops as the test group. Unoprostone is the main ingredient of a Rescula ophthalmic solution, and unoprostone eye drops were administered (two drops once per day, per eye) with Rescula ophthalmic solution 0.12% (Nitto Medic Co., Ltd., 1 mL contains 1.2 mg of isopropyl unoprostone). The refractive index and the axial length of the mouse eyeball after three weeks were measured and the amounts of change were calculated. The refractive index measurement and the axial length measurement were the same as in Experiment 1.

### (Results)

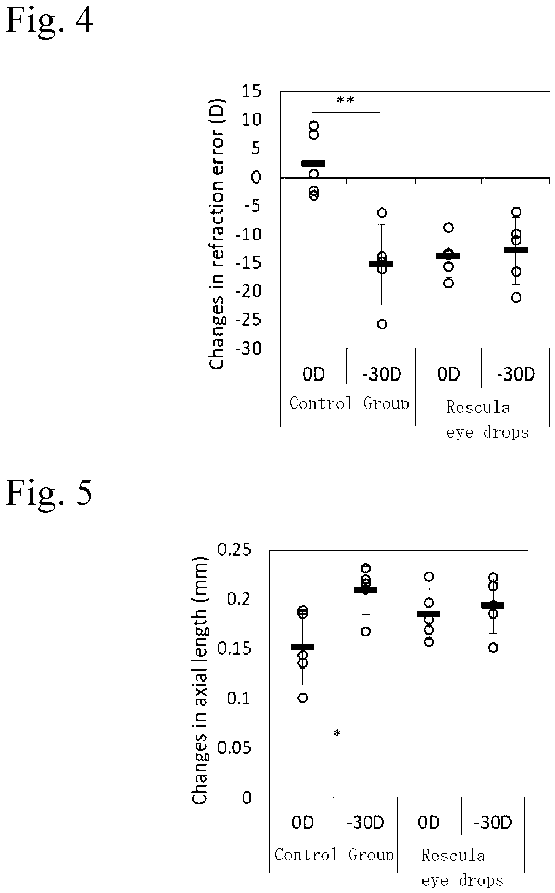

Fig. 4 shows the refractive index results. As the changes in refractive index in the control group, it was confirmed that the eyes with the -30D lenses became myopic and were significantly different from the eyes with 0D lenses. On the other hand, in the eye drop group, both eyes became myopic and no myopia suppressing effect of the eye drops was confirmed.

Fig. 5 shows the axial length results. As the changes in axial length in the control group, it was confirmed that the axial lengths of the eyes with the -30D lenses elongated and were significantly different from the axial lengths of the eyes with 0D lenses. On the other hand, in the eye drop group, no significant difference in the axial lengths of the two eyes was confirmed.

From the above results, it was found that an ophthalmic solution containing unoprostone does not exhibit a myopia suppressing effect.

## Claims

1. An ophthalmic preparation for myopia suppression, the ophthalmic preparation comprising:
bunazosin.

2. An ophthalmic preparation for myopia suppression, the ophthalmic preparation comprising:
a composition containing bunazosin.

3. A method of manufacturing an ophthalmic preparation for myopia suppression, the method comprising:
using a composition containing bunazosin.

4. A method of suppressing myopia, the method comprising:
administering a composition containing bunazosin as an ophthalmic preparation.
